# EUROPEAN PATENT APPLICATION

(11) **EP 4 826 926 A1**
(43) Date of publication of application: **21.10.2026**
(21) Application number: 24903700.3
(22) Date of filing: 11.12.2024
(51) Int. Cl.: H01S 5/02208, A61B 18/24, H01S 5/0225, H01S 5/0233, H01S 5/024, H01S 5/183

(54) **LIGHT RADIATION DEVICE AND IN-VIVO LIGHT RADIATION ASSEMBLY USING SAME**

(30) Priority: 15.12.2023 JP 2023211659
(71) Applicant: NICHIA CORPORATION, Anan-shi, Tokushima 774-8601 (JP)
(72) Inventor: MIYATA, Tadaaki, Anan-shi, Tokushima 774-8601 (JP)
(74) Representative: Betten & Resch
(86) International application number: PCT/JP2024/043717
(87) International publication number: WO 2025/127046

(57) **Abstract**

To provide a small light irradiation device having heat dissipation and electrical insulation properties. The light irradiation device includes: a light-emitting element configured to emit light having a predetermined wavelength; a support body on which the light-emitting element is mounted; insulated wires electrically connected to the light-emitting element; a case in which the light-emitting element, connection portions each between a respective one of the insulated wires and the light-emitting element, and a part of the support body are accommodated; and a sealing member having an insulation property and sealing the light-emitting element, the part of the support body, and the connection portions inside the case, in which the other part of the support body protrudes from the sealing member toward an outside of the case.

## Description

### Technical Field

The present disclosure relates to a light irradiation device and an in-vivo light irradiation assembly using the same.

### Background Art

Endoscopes are used for diagnoses and treatments in a biological body. A treatment using a combination of an endoscope and laser irradiation is also performed. In photoimmunotherapy that has been attracting attention in recent years, when an affected area is on the body surface or in a region shallow from the body surface, the body surface is irradiated with light having a specific wavelength that reacts with a photosensitizer accumulated in cancer cells. When the affected area is located at a position deep from the body surface, the affected area in the body is irradiated with laser light by puncturing. Specifically, an optical fiber is inserted into a blood vessel, a digestive tract, or other lumen, and the affected area in the biological body is irradiated with laser light having a specific wavelength. A deployable balloon illumination for endoscopy has been proposed (for example, see Patent Document 1).

### Citation List

### Patent Literature

Patent Document 1: JP 2022-535144 T

### SUMMARY OF INVENTION

### Technical Problem

The configuration using the optical fiber is weak against bending, and optical loss increases in proportion to the length of the optical fiber. An ultra-small laser light source that can be inserted into the vicinity of an affected area in a body without an optical fiber is desired. According to one aspect of the present disclosure, a small light irradiation device having heat dissipation and electrical insulation properties can be provided.

### Solution to Problem

In one embodiment, a light irradiation device includes: a light-emitting element configured to emit light having a predetermined wavelength; a support body on which the light-emitting element is mounted; insulated wires electrically connected to the light-emitting element; a case accommodating the light-emitting element, connection portions each between a respective one of the insulated wires and the light-emitting element, and a part of the support body; and a sealing member having an insulation property and sealing the light-emitting element, the part of the support body, and the connection portions inside the case, wherein the other part of the support body protrudes from the sealing member toward an outside of the case.

### Advantageous Effects of Invention

A small light irradiation device having heat dissipation and electrical insulation properties is implemented.

### BRIEF DESCRIPTION OF DRAWINGS

FIG. 1 is a diagram illustrating an example of an in-vivo light irradiation assembly using a light irradiation device of a first embodiment.
FIG. 2A is a schematic view of the light irradiation device of the first embodiment.
FIG. 2B is a view illustrating another example of a sealed state of the light irradiation device in FIG. 2A.
FIG. 3A is a side view illustrating the appearance of a light-emitting element mounting portion of the light irradiation device in FIGS. 2A and 2B.
FIG. 3B is a schematic view illustrating a configuration example of an optical component.
FIG. 4 is a schematic view of a light-emitting element mounted on a support body.
FIG. 5 is a schematic view illustrating the sealed state of the light irradiation device.
FIG. 6 is a schematic view illustrating an example in which the support body and a light-emitting element are layered.
FIG. 7 is a diagram illustrating an example of an in-vivo light irradiation assembly using a light irradiation device of a second embodiment.
FIG. 8 is a schematic view of the light irradiation device of the second embodiment.
FIG. 9 is a schematic view of a light irradiation device of a third embodiment.
FIG. 10 is a schematic view illustrating an example of a light-emitting element used in the third embodiment.
FIG. 11A is a perspective view of a support body used in the third embodiment on a light-emitting element mounting surface side.
FIG. 11B is a perspective view of the support body used in the third embodiment on a back surface side.

### DESCRIPTION OF EMBODIMENTS

Hereinafter, an embodiment for carrying out the present disclosure is described with reference to the drawings. The following description is intended to embody technical concepts of the present disclosure, and the present disclosure is not limited to the following unless specifically stated. In each drawing, members having identical functions may be denoted by the same reference characters. In consideration of the ease of explanation or understanding of the gist, the embodiments may be illustrated separately for convenience, but the partial substitutions or combinations of the configurations illustrated in different embodiments and examples are possible. In the embodiments to be described below, configurations different from the embodiment described before will be mainly described, and redundant descriptions of that are common with the embodiment described before will sometimes be omitted. The size, positional relationship, and other features of members illustrated in the drawings may be exaggerated in order to clarify explanation.

### First Embodiment

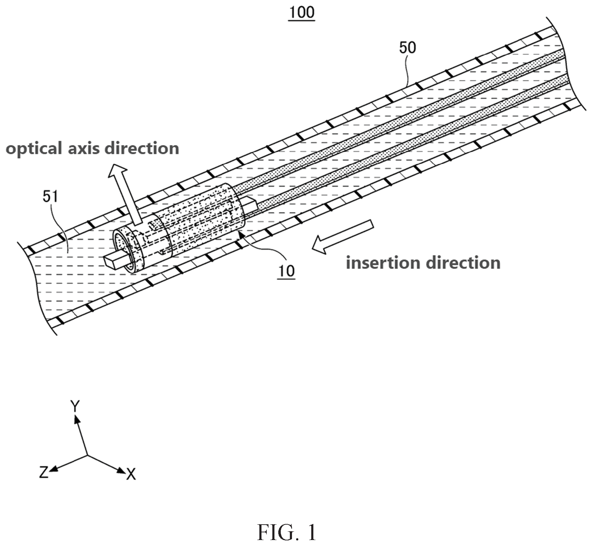

FIG. 1 is a diagram illustrating an example of an in-vivo light irradiation assembly 100 using a light irradiation device 10 of a first embodiment. The in-vivo light irradiation assembly 100 includes a catheter 50 and the light irradiation device 10 inserted into the catheter 50, and the inside of the catheter 50 is filled with a coolant 51 at least during use. The in-vivo light irradiation assembly 100 is used for treatment, diagnosis, sensing, and the like. The light irradiation device 10 is inserted into the catheter 50 in an "insertion direction" indicated by a white arrow in the drawing, for example, and is introduced into a biological body. Because the light irradiation device 10 is a heat generating element, the light irradiation device 10 is used in a state in which the coolant 51 is supplied into the catheter 50 and a heat generating portion is cooled. The coolant 51 is, for example, physiological saline, blood, lymph, or the like, and the coolant temperature is, for example, about 10 °C to 36 °C. Because the light irradiation device 10 is inserted into the vicinity of a target site such as an affected area in the biological body by using the catheter 50, the affected area such as a tumor can be directly irradiated with light without using an optical fiber, or the target site for diagnosis, sensing, or the like can be examined and confirmed.

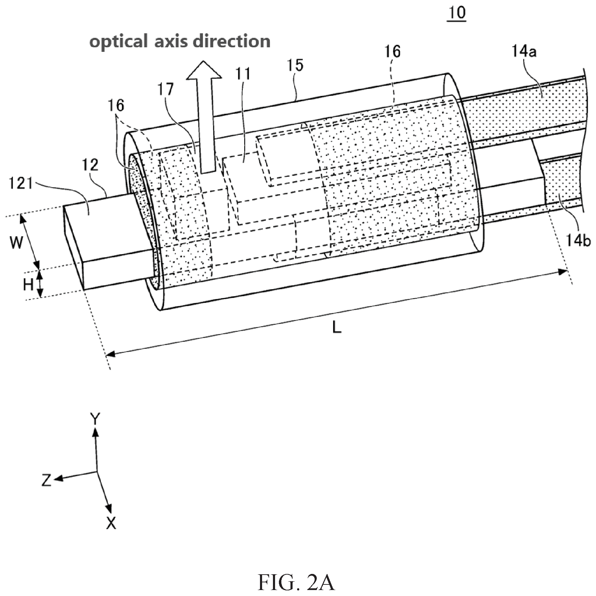

FIG. 2A is a schematic view of the light irradiation device 10. The light irradiation device 10 includes a light-emitting element 11 that emits light having a predetermined wavelength, a support body 12 on which the light-emitting element 11 is mounted, and insulated wires 14a and 14b electrically connected to the light-emitting element 11. Each of the insulated wires 14a and 14b includes a metal wire and an insulating coating. The metal wire is covered with the insulating coating. The light irradiation device 10 further includes a case 15 and an insulating sealing member 16 sealing the case 15. The case 15 accommodates therein the light-emitting element 11, a part of the support body 12, and a connection portion between the light-emitting element 11 and the insulated wire 14a and a connection portion between the light-emitting element 11 and the insulated wire 14b. The sealing member 16 seals the light-emitting element 11, the part of the support body 12, and the connection portion between the light-emitting element 11 and the insulated wire 14a and the connection portion between the light-emitting element 11 and the insulated wire 14b inside the case 15. Another part of the support body 12 protrudes from the sealing member 16 to the outside of the case 15. At least a light-emitting portion of the case 15 is light transmissive.

In the coordinate system of FIG. 2A, an optical axis direction is defined as a Z direction, a direction in which the light-emitting element 11 is mounted on the support body 12 is defined as a Y direction, and a direction orthogonal to the Z direction and the Y direction is defined as an X direction. In this configuration example, the longitudinal direction of the support body 12 is parallel to the Z direction. The light irradiation device 10 of the first embodiment is inserted into the catheter 50 in the + Z direction, and the insulated wires 14a and 14b extend in the - Z direction. The support body 12 is made of an insulating material such as silicon (Si), aluminum nitride (AlN), silicon nitride (SiN), sapphire (Al₂O₃), glass, quartz, or ceramic, and the light-emitting element 11 is mounted on a light-emitting element mounting surface 121 of the support body 12.

As one of the features of the light irradiation device 10, a length L of the support body 12 is greater than the length of the case 15 in the Z direction, and at least one of a tip end side (+ Z side) or a rear end side (- Z side) of the support body 12 protrudes from the sealing member 16 to the outside of the case 15. As illustrated in FIG. 2A, with the connection portion between the light-emitting element 11 and the insulated wire 14a and the connection portion between the light-emitting element 11 and the insulated wire 14b accommodated inside the case 15, both ends of the support body 12 in the longitudinal direction (Z direction) may protrude from the case 15, and then the case 15 may be sealed with the sealing member 16. With the at least one of both ends of the support body 12 in the longitudinal direction protruded toward the outside of the sealing member 16, the support body 12 is brought into contact with the coolant 51 to cool the light irradiation device 10, and the coolant 51 is prevented from entering the case 15.

The case 15 is a transparent cylindrical body made of glass, quartz, biocompatible plastic, or the like. The outer diameter of the case 15 is, for example, in a range from 0.4 mm to 2.0 mm, and the inner diameter thereof is in a range from 0.3 mm to 1.5 mm. As an example, a biocompatible plastic tube with an outer shape of 0.5 mm and an inner diameter of 0.38 mm is used. A width W of the support body 12 is smaller than the inner diameter of the case 15, but may be set as great as possible within a range in which the support body 12 can be accommodated in the case 15 in consideration of increasing the contact area with the coolant 51. The support body 12 has a height H, that is, a thickness that allows the support body 12 to stably support the light-emitting element 11. The height H is, for example, about 0.1 mm to 0.3 mm. The length L of the support 12 is greater than the length of the case 15 in the cylindrical axis direction, and is determined according to the length of the case 15. When the length of the case 15 in the cylindrical axis direction is 1.0 mm, the length L of the support body 12 is, for example, in a range of 1.3 mm to 3.0 mm.

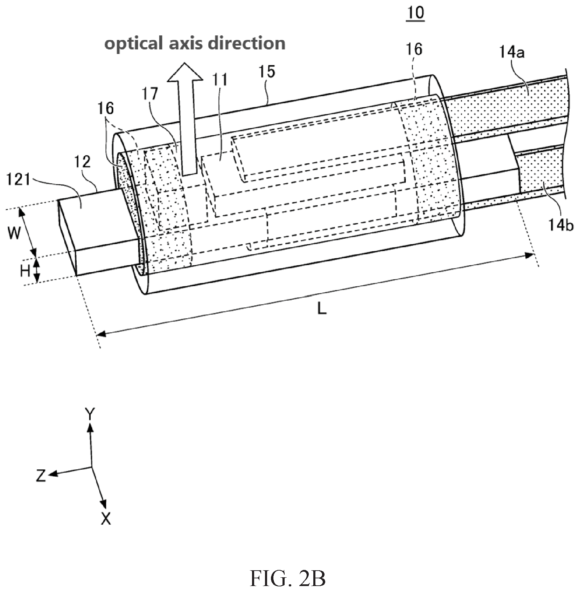

The sealing member 16 sealing both ends of the case 15 along the longitudinal direction of the support body is an insulating resin sealing member, and prevents the coolant 51 from entering the case 15. As the resin sealing member, an epoxy resin, a silicone resin, an acrylic resin, a thermoplastic resin, or the like can be used. A biocompatible insulating resin may be used as the sealing member 16. Insulating resins that are biocompatible and have high thermal conductivity are more preferably used for the sealing member 16. As such a resin material, polycarbonate (PC), polyethylene terephthalate (PET), polyimide (PI), polyurethane, polyester imide, or the like, whose components and composition are adjusted to mitigate adverse effects on a biological body, can be used. The sealing member 16 seals the electrical connection portion between the light-emitting element 11 and the insulated wire 14a and the electrical connection portion between the light-emitting element 11 and the insulated wire 14b inside the case 15. In the example of FIG. 2A, from the viewpoint of efficiently releasing heat generated from the light-emitting element 11, a part of the light-emitting element 11 is covered with the sealing member 16 on the rear end side of the case 15; however, the light-emitting element 11 may not necessarily be in contact with the sealing member 16 as long as the coolant 51 can be prevented from entering the case 15. FIG. 2B illustrates another example of the sealed state of the light irradiation device 10. The sealing member 16 seals the case 15 without being in contact with the light-emitting element 11 on the rear end side of the case 15. In this case, the light-emitting element 11 is accommodated in an internal space of the case 15 without contacting the sealing member 16. The insulated wires 14a and 14b are extended outward of the case 15 from at least one end portion of the case 15 while maintaining the sealed state of FIG. 2A or FIG. 2B. In the configuration examples of FIGS. 2A and 2B, the insulated wires 14a and 14b are extended outward from the rear end of the case 15 in the - Z direction so as not to hinder the light emission from the light-emitting element 11 in the Y direction.

The insulated wires 14a and 14b extending out of the case 15 are cooled by the coolant 51 and also function as a heat dissipation material. The insulated wires 14a and 14b are, for example, enameled wires. A metal wire of a good conductor such as Cu or Ni is insulation-coated with an insulating coating of polyurethane. Instead of polyurethane, coating of polyester, polyesterimide, polyamideimide, polyimide, or the like, which is an insulating resin, may be applied. The insulating coating, which is the surface coating on the insulated wires 14a and 14b, is used as a heat dissipation material to suppress accumulation of heat inside the case 15. When rectangular enameled wires are used as the insulated wires 14a and 14b, the wires are wide wires, and the bonding area with the support body 12 can be increased. In addition, when the cross-sectional area is equal, the rectangular enameled wire can be made thinner than a round enameled wire, and thus can be made smaller in size.

The light-emitting element 11 is, for example, an edge-emitting laser element, and resonates in a direction parallel to a Z axis. When an edge-emitting laser element is used as the light-emitting element 11, one end surface thereof in the resonance direction serves as a light-emitting surface. An optical component 17 is disposed inside the case 15 to face the light-emitting surface of the light-emitting element 11, and causes light emitted from the edge-emitting laser element to travel in a direction intersecting the light-emitting element mounting surface 121. The direction intersecting the light-emitting element mounting surface 121 is any of all directions except for the direction parallel to the light-emitting element mounting surface 121, but may be, for example, in a range of 50° to 130° relative to the light-emitting element mounting surface 121. It may be a direction perpendicular to the light-emitting element mounting surface 121. The expression "perpendicular" direction does not need to be exactly a right angle to the light-emitting element mounting surface 121, and includes a range of 90° ± 5° in consideration of manufacturing errors and surface states of the support body 12 and the optical component 17. The emitted light from the light-emitting element 11 is reflected by the optical component 17 and is extracted to the outside of the case 15. The optical component 17 is a mirror or prism having a reflective surface. As the optical component 17, a meta-polarization element having a thin metasurface element inserted therein may be used. The optical component 17 may be an optical element having a lens function of controlling the divergence angle of the light emitted from the light-emitting element 11.

The insulated wires 14a and 14b are arranged such that they do not obstruct the laser light output described above. The insulated wire 14a is electrically connected directly or indirectly to one of the electrodes of the laser element on the side of the light-emitting element mounting surface 121 of the support body 12. The other insulated wire 14b is electrically connected to the other electrode of the laser element on the back surface (the surface on the opposite side to the light-emitting element mounting surface 121) of the support body 12. The insulated wires 14a and 14b are extended outward of the case 15 from the side opposite to the light-emitting surface of the laser element. The insulated wire 14a electrically connected to the light-emitting element on the side of the light-emitting element mounting surface 121 is also referred to as a first insulated wire. The insulated wire 14b electrically connected to the light-emitting element on the surface of the support body 12 opposite to the light-emitting element mounting surface 121 is also referred to as a second insulated wire.

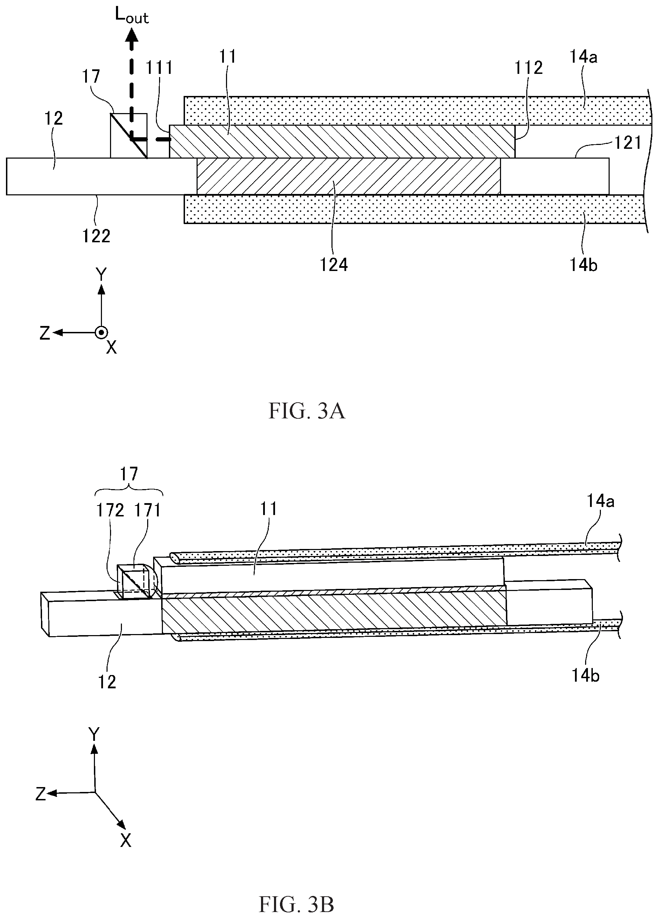

FIG. 3A is a side view illustrating the appearance of a light-emitting element mounting portion of the light irradiation device 10 in FIGS. 2A and 2B. FIG. 3A illustrates the configuration in a state before the light-emitting element mounted portion is inserted into the light-transmissive case 15 and sealed with the sealing member 16. The surface of the light-emitting element 11 facing the optical component 17 is a light-emitting surface 111. A rear end surface 112 on the opposite side to the light-emitting surface is a reflective surface, and a highly reflective film may be formed thereon. A laser cavity is formed between the light-emitting surface 111 and the rear end surface 112. Emitted light from the light-emitting element 11 is reflected by the optical component 17, and output light Lout of the light irradiation device 10 is extracted in the direction of the broken line arrow.

A part of the insulating coating on the insulated wire 14a is removed, and the inside metal wire is connected to one of the electrodes of the light-emitting element 11. A part of the insulating coating on the insulated wire 14b is removed, and the inside metal wire is electrically connected to the other electrode of the light-emitting element 11. In the example of FIG. 3A, a conductive film 124 is formed from the light-emitting element mounting surface 121 of the support body 12 to a back surface 122 through the lateral surface, and the metal wire inside the insulated wire 14b is connected to the conductive film 124. This allows for establishing electrical connection between the other electrode of the light-emitting element 11 and the insulated wire 14b. With the conductive film 124 for electrical connection provided on the surface of the support body 12, the conductive film 124 can be used as a heat dissipation member.

FIG. 3B is a schematic view illustrating a configuration example of the optical component 17. FIG. 3B illustrates a structure before being accommodated in the case 15. The optical component 17 may include two or more optical elements. For example, the optical component 17 may include an optical element 171 having a lens function of controlling the divergence angle of the light emitted from the light-emitting element 11, and an optical element 172 that reflects, at a predetermined angle or in a predetermined direction, the light whose divergence angle has been controlled by the optical element 171. In this configuration example, the incident surface of the optical element 171 is formed into a convex surface, but the reflective surface of the optical element 172 may be formed into a concave surface. By integrating a plurality of optical elements 171 and 172 to form the optical component 17, the size of the entire optical component 17 can be reduced, and the light irradiation device 10 can be downsized accordingly.

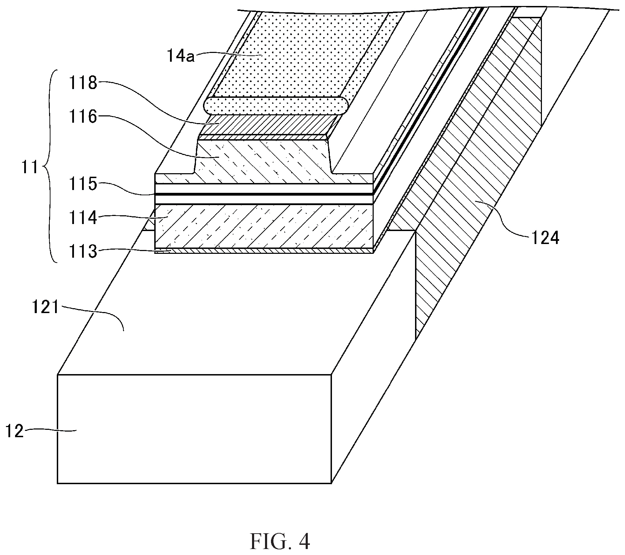

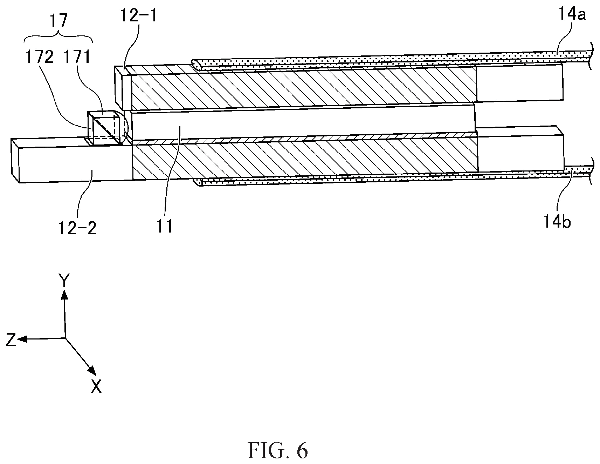

FIG. 4 is a schematic view of the light-emitting element 11 mounted on the support body 12. The light-emitting element 11 is, for example, an edge-emitting laser element having a ridge structure. The light-emitting element 11 includes an n-side electrode 113, an n-side semiconductor layer 114, an active layer 115, a p-side semiconductor layer 116, and a p-side electrode 118. The n-side semiconductor layer 114 may include an optical guide layer, a cladding layer, a contact layer, and the like doped with an n-type impurity. The p-side semiconductor layer 116 may include an optical guide layer, a cladding layer, a contact layer, and the like doped with a p-type impurity. The p-side electrode 118 is electrically connected to the insulated wire 14a. When an ITO electrode is used as the p-side electrode 118, the p-side electrode 118 may also serve as a cladding layer. The n-side electrode 113 is electrically connected to the other insulated wire 14b via the conductive film 124 formed on the support body 12. In the example of FIG. 4, the p-side electrode 118 and the insulated wire 14a are directly connected, but may be indirectly connected. For example, a second support body may be disposed between the p-side electrode 118 and the insulated wire 14a. As described below with reference to FIG. 6, the heat dissipation property can be improved by sandwiching the light-emitting element 11 between two support bodies 12.

The semiconductor materials of the light-emitting element 11 and their composition are designed to emit laser light having a desired wavelength. When the light-emitting element 11 is used as an ultraviolet laser, a violet laser, a blue laser, and a green laser, a GaN-based material such as GaN, InGaN, and AlGaN is used. When the light-emitting element 11 is used as a red laser, an infrared laser, and a near-infrared laser, a GaAs-based material such as GaAs or AlGaAs or an InP-based material such as InAlGaP or GaInP is used. From the viewpoint of sealing the light-emitting element 11 inside the case 15, the resonance length of the laser element may be short, and the ridge may be widened to secure the gain of the active layer 115. The width of the ridge may be designed to be, for example, 2 µm to 100 µm. The transverse mode may be a multi-mode or a single mode.

Unlike a fiber-type or external light source-type irradiation device, the light emitted by the light-emitting element 11 is linearly polarized light, and is reflected at the optical component 17 such as a mirror to travel in a different direction, so that the direction of the linearly polarized light after emission can be changed. This allows for reducing the reflectance of light incident on a biological body and improving the transmittance of light to the biological body, for example. Even if laser light is in a specific polarization state upon being emitted, its polarization state may fluctuate or it may be depolarized, while propagating through a transmission medium such as an optical fiber. In the present embodiment, since the affected area in the body can be directly irradiated with light without using an optical fiber, a light can be irradiated to a target portion such as the affected area while maintaining a specific polarization state.

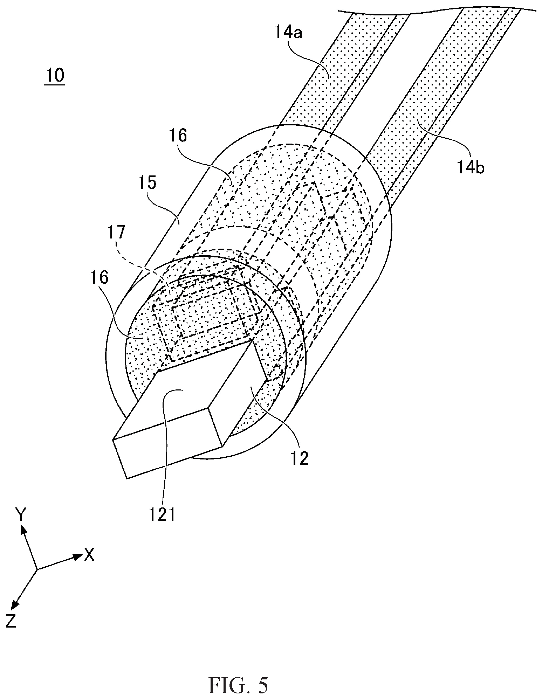

FIG. 5 is a schematic view illustrating a sealed state of the case 15. The optical component 17 is disposed on the light-emitting element mounting surface 121 of the support body 12 to face the light-emitting surface 111 of the light-emitting element 11, and a portion of the support body 12 where the light-emitting element 11 is disposed is inserted into the case 15. The light irradiation device 10 is obtained by sealing both ends of the case 15 with the sealing member 16 in a state in which at least one of both ends of the support body 12 in the longitudinal direction protrudes toward the outside of the case 15. The support body 12 protruding from the case 15 is in contact with the coolant 51 (see FIG. 1), so that the heat dissipation property of the light irradiation device 10 is ensured. In addition, the surfaces of the insulated wires 14a and 14b are used as a heat dissipation member, so that the heat dissipation property is improved. By sealing both ends of the case 15 with the sealing member 16, a short circuit due to the coolant 51 can be prevented. The overall thickness and width of the light irradiation device 10 including the case 15 are reduced to about 0.5 mm, and an ultra-small light irradiation device 10 that can be incorporated into the catheter 50 is realized.

FIG. 6 is a schematic view illustrating an example in which the support body 12 and the light-emitting element 11 are layered. In this configuration example, the light-emitting element 11 is arranged between a first support body 12-1 and a second support body 12-2. The first support body 12-1 is in contact with the upper surface of the light-emitting element 11, and the second support body 12-2 is in contact with the bottom surface of the light-emitting element 11. By arranging the light-emitting element 11 between the first support body 12-1 and the second support body 12-2, heat can be efficiently released to the upper side and the lower side of the light-emitting element 11, and the heat dissipation property of the light irradiation device 10 can be improved. The rear end of at least one of the first support body 12-1 or the second support body 12-2 may extend in the - Z direction. In FIG. 6, it is assumed that light is extracted by the optical component 17 in a direction (for example, the Y direction) not parallel to the optical axis (Z axis); however, as will be described in a second embodiment, when light is extracted in a direction parallel to the light-emitting element mounting surface 121 of the support body 12, the tip end sides of the first support body 12-1 and the second support body 12-2 may be extended long in the + Z direction.

### Second Embodiment

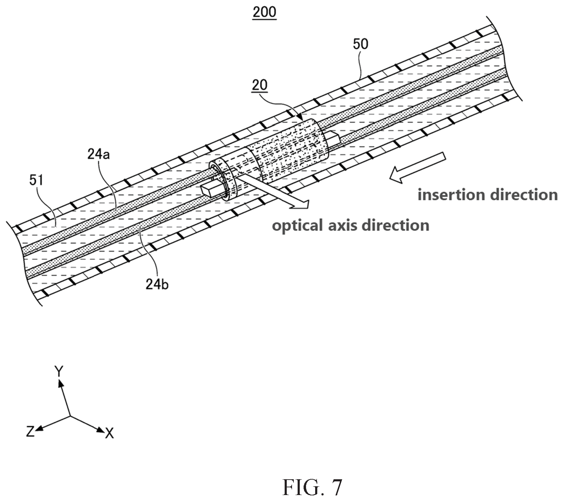

FIG. 7 is a diagram illustrating an example of an in-vivo light irradiation assembly 200 using a light irradiation device 20 of the second embodiment. The in-vivo light irradiation assembly 200 includes the catheter 50 and the light irradiation device 20 inserted into the catheter 50, and the inside of the catheter 50 is filled with the coolant 51 at least during use. The in-vivo light irradiation assembly 200 is used for treatment, diagnosis, sensing, and the like. Similarly to the light irradiation device 10 of the first embodiment, the light irradiation device 20 is inserted into the catheter 50 in the "insertion direction" indicated by a white arrow in the drawing, and is introduced into a biological body. The inside of the catheter 50 is filled with the coolant 51. The light irradiation device 20 is used in a state in which the heat generating portion thereof is cooled. In the second embodiment, insulated wires 24a and 24b are extended outward from both ends of case 15 to the outside of the case to enhance the heat dissipation property. As the lengths of the portions of each of the insulated wires 24a and 24b extended outward from both ends of the case 15 to the outside of the case, the length of the portion on a side opposite to the light-emitting surface side of the light-emitting element 11 may be greater than the length of the portion on the light-emitting surface side of the light-emitting element 11. In order to realize this configuration, light emitted from the light-emitting element 11 is guided in a direction parallel to a light-emitting element mounting surface or in a direction in which the light is not interfered with by the insulated wires 24a and 24b.

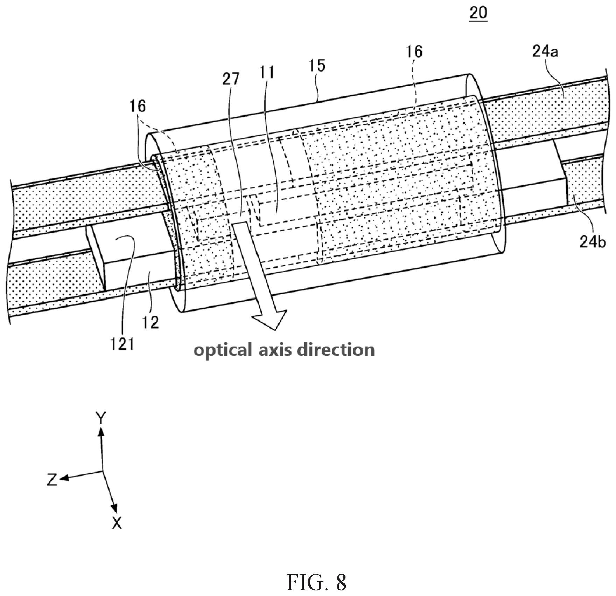

FIG. 8 is a schematic view of the light irradiation device 20. The light irradiation device 20 includes the light-emitting element 11 that emits light having a predetermined wavelength, the support body 12 on which the light-emitting element 11 is mounted, and the insulated wires 24a and 24b electrically connected to the light-emitting element 11. The light irradiation device 20 further includes the case 15 and the insulating sealing member 16 sealing the case 15. The case 15 accommodates therein the light-emitting element 11, a part of the support body 12, and a connection portion between the light-emitting element 11 and the insulated wire 24a and a connection portion between the light-emitting element 11 and the insulated wire 24b. The sealing member 16 seals the light-emitting element 11, the part of the support body 12, and the connection portion between the light-emitting element 11 and the insulated wire 24a and the connection portion between the light-emitting element 11 and the insulated wire 24b inside the case 15. One or both of the end portions of the support body 12 in the longitudinal direction protrude from the sealing member 16 toward the outside of the case 15. At least a light-emitting portion of the case 15 is light transmissive.

One insulated wire 24a is electrically connected to the light-emitting element 11 on the side of the light-emitting element mounting surface 121 of the support body 12. The other insulated wire 24b is electrically connected to the light-emitting element 11 on the back surface (the surface on the opposite side to the light-emitting element mounting surface 121) of the support body 12. The insulated wires 24a and 24b are disposed with the light-emitting element 11 and the support body 12 interposed between the insulated wires 24a and 24b, similarly to the first embodiment.

The light-emitting element 11 is, for example, an edge-emitting laser element. The light-emitting element 11 may be a laser element having a wide ridge structure that is the same as or similar to that of the first embodiment. The direction parallel to the Z-axis of the light-emitting element 11 is the resonance direction. An optical component 27 is disposed inside the case 15, and causes light emitted from the light-emitting surface of the laser element to travel in a direction parallel to the light-emitting element mounting surface 121 of the support body 12. Since the insulated wires 24a and 24b extend in the + Z direction and the - Z direction with the light-emitting element 11 and the support body 12 interposed between the insulated wires 24a and 24b, the light from the light-emitting element 11 is extracted to the outside of the case 15 in such a direction that the light is not interfered with by the insulated wires 24a and 24b.

The optical component 27 reflects the light emitted from the light-emitting surface of the light-emitting element 11 in, for example, the X direction parallel to the light-emitting element mounting surface 121. The expression "parallel" direction does not need to be exactly parallel to the light-emitting element mounting surface 121, and may have an error of about ± 10° including a surface state of the support body 12, a manufacturing error of the optical component 27, and the like. The laser light reflected by the optical component 27 may be extracted at any angle other than parallel as long as the laser light is not interfered with by the insulated wires 24a and 24b. The optical component 27 is preferably as thick as the light-emitting element 11 or thinner than the light-emitting element 11. The optical component is a mirror or prism flattened to be thin, or a meta-polarization element having a thin metasurface element inserted therein. The optical component 27 may be an optical element having a lens function of controlling the divergence angle of the light emitted from the light-emitting element 11. The optical component 27 may include two or more optical elements as described above with reference to FIG. 3B. For example, the optical component 27 may include an optical element having a lens function of controlling the divergence angle of the light emitted from the light-emitting element 11, and an optical element that reflects, at a predetermined angle, the light whose divergence angle has been controlled by the optical element.

The sealing member 16 seals the case 15 in a state in which at least one of both ends of the support body 12 protrudes toward the outside of the case 15, and the insulated wires 24a and 24b are extended outward from both ends of the case 15 to the outside of the case 15. The electrical connection portion between the light-emitting element 11 and the insulated wire 24a, the electrical connection portion between the light-emitting element 11 and the insulated wire 24b, and the optical component 27 are sealed inside the case 15 and are isolated from the coolant 51. The sealing member 16 is preferably a biocompatible resin having high thermal conductivity. As illustrated in FIG. 2B, the sealing member 16 may seal the rear end portion of the case 15 so as not to come into contact the light-emitting element 11 on the rear end side of the case 15.

The support body 12 protruding from the case 15 is in contact with the coolant 51, so that the heat dissipation property of the light irradiation device 20 is ensured. In addition, with the insulated wires 24a and 24b extended outward of both sides of the case 15 in the longitudinal direction, the heat dissipation property is further improved as compared with the first embodiment. Similarly to the first embodiment, the overall thickness and width of the light irradiation device 20 including the case 15 are about 0.5 mm, and an ultra-small light irradiation device 20 that can be incorporated into the catheter 50 is realized. In the configuration of the second embodiment, the light-emitting element 11 may be arranged between two support bodies 12-1 and 12-2 as illustrated in FIG. 6. In the second embodiment, since the light emitted from the light-emitting element 11 is extracted in a direction in which the light is not obstructed by the support body 12, the heat dissipation property of the light irradiation device 20 can be further improved by arranging the light-emitting element 11 between the two support bodies 12-1 and 12-2.

### Third Embodiment

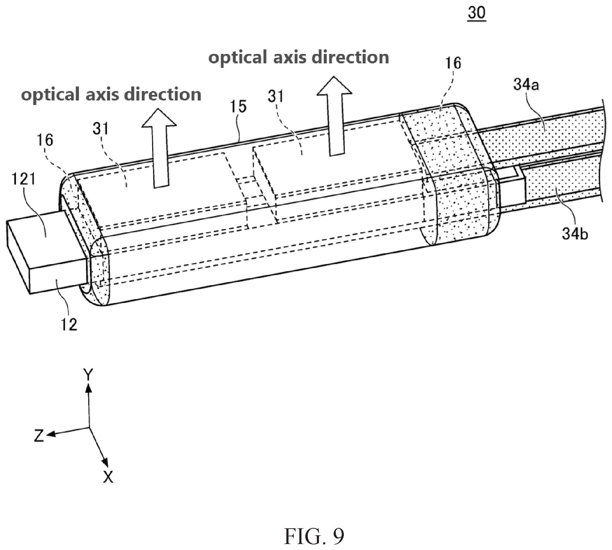

FIG. 9 is a schematic view of a light irradiation device 30 of the third embodiment. Similarly to the first embodiment and the second embodiment, the light irradiation device 30 is also used by being incorporated into the catheter 50 (see FIGS. 1 and 7), and the light irradiation device 30 and the catheter 50 can constitute an in-vivo light irradiation assembly. In the third embodiment, a vertical cavity surface emitting laser (VCSEL) is used as a light-emitting element 31.

The light irradiation device 30 includes two light-emitting elements 31 that emit light having a predetermined wavelength, the support body 12 on which the light-emitting elements 31 are mounted, and insulated wires 34a and 34b electrically connected to the light-emitting elements 31. The light irradiation device 30 further includes the case 15 and the insulating sealing member 16 sealing the case 15. The case 15 accommodates therein the light-emitting elements 31, a part of the support body 12, connection portions each between the insulated wire 34a and a respective one of the light-emitting elements 31, and connection portions each between the insulated wire 34b and a respective one of the light-emitting elements 31. The sealing member 16 seals the light-emitting elements 31, the part of the support body 12, the connection portions each between the insulated wire 34a and a respective one of the light-emitting elements 31, and the connection portions each between the insulated wire 34b and a respective one of the light-emitting elements 31 inside the case 15. The other part of the support body 12 protrudes from the sealing member 16 toward the outside of the case 15. At least a light-emitting portion of the case 15 is light transmissive.

The light-emitting surface of the VCSEL used as the light-emitting element 31 faces the case 15 in a direction perpendicular to the light-emitting element mounting surface 121 of the support body 12. Since the laser light emitted from the VCSEL passes through the case 15 in the Y direction perpendicular to the light-emitting element mounting surface 121 as indicated by the white arrow in the drawing, an optical component such as a mirror is not required. The sealing member 16 sealing both ends of the case 15 is provided in a range in which light emission from the VCSEL is not hindered.

The insulated wires 34a and 34b are, for example, two-core enameled wires. One insulated wire 34a is electrically connected to the light-emitting elements 31 on the side of the light-emitting element mounting surface 121 of the support body 12. The other insulated wire 34b is electrically connected to the light-emitting elements 31 on the back surface (the surface on the opposite side to the light-emitting element mounting surface 121) of the support body 12. The insulated wires 34a and 34b are extended outward from at least one of both ends of the case 15 in the longitudinal direction to the outside of the case 15 with the support body 12 interposed between the insulated wires 34a and 34b.

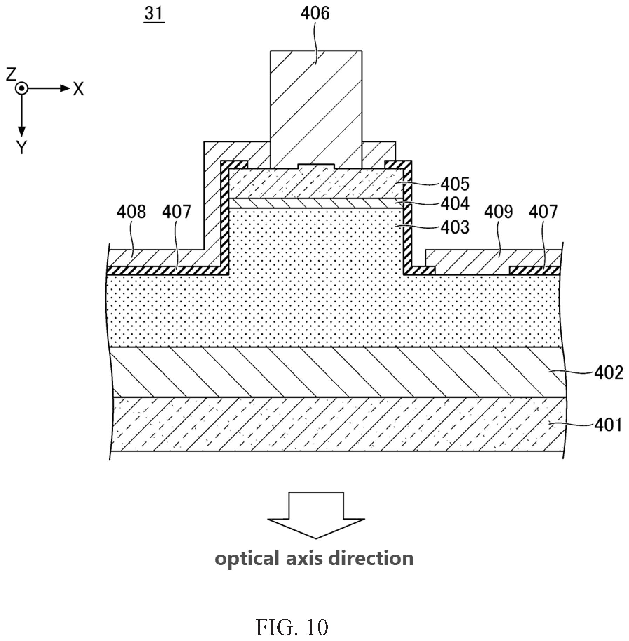

FIG. 10 is a schematic view illustrating an example of the light-emitting element 31 used in the third embodiment. The VCSEL as the light-emitting element 31 has a layered structure in which a semiconductor substrate 401, an n-side reflective film 402, an n-type semiconductor layer 403, an active layer 404, a p-type semiconductor layer 405, and a p-side reflective film 406 are layered in this order in the - Y direction. The light emission direction is the + Y direction. The conductivity types of the p-type and the n-type may be reversed. The semiconductor substrate 401 may be removed. The n-type semiconductor layer 403 includes a flat plate portion and a protruding portion protruding from the flat plate portion in the - Y direction. The active layer 404 is provided on an upper surface of the protruding portion of the n-type semiconductor layer 403. The p-type semiconductor layer 405 is provided on an upper surface of the active layer 404, and the p-side reflective film 406 is provided in a region of an upper portion of the p-type semiconductor layer 405, other than the peripheral region of the upper portion. A p-side contact layer may be provided between the p-type semiconductor layer 405 and the p-side reflective film 406.

The light-emitting element 31 includes an insulating layer 407 that covers an upper surface of the flat plate portion and a lateral surface of the protruding portion of the n-type semiconductor layer 403, a lateral surface of the active layer 404, and a lateral surface and a peripheral region of an upper surface of the p-type semiconductor layer 405. The light-emitting element 31 includes a p-side electrode 408 electrically connected to the p-type semiconductor layer 405, and an n-side electrode 409 electrically connected to the n-type semiconductor layer 403. The side on which the P-side electrode 408 and the n-side electrode 409 are provided is disposed on the light-emitting element mounting surface 121 of the support body 12. A conductive connection layer (or bump) to be connected to the light-emitting element mounting surface 121 of the support body 12 may be provided so that the height of the p-side electrode 408 and the n-side electrode 409 is aligned in the - Y direction. When the light-emitting element 31 is flip-chip mounted on the support body 12 with the conductive connection layer connected to the p-side electrode 408 and the n-side electrode 409, the p-side reflective film 406 does not interfere with the light-emitting element mounting surface of the support body 12.

The n-side reflective film 402 and the p-side reflective film 406 may each be formed of, for example, a distributed Bragg reflector (DBR). The DBR has a structure in which a plurality of high refractive index layers and a plurality of low refractive index layers are alternately layered. The DBR includes a wavelength region of high reflectance called a stop band. A center wavelength and a wavelength width of the stop band are determined by a refractive index and a thickness of the high refractive index layer and a refractive index and a thickness of the low refractive index layer. The reflectance in the stop band of the DBR increases with the refractive index difference between the high refractive index layer and the low refractive index layer and with the number of layers.

In the example illustrated in FIG. 10, a standing wave is formed between the n-side reflective film 402 and the p-side reflective film 406. A wavelength of the standing wave in the atmosphere is within the stop bands of the n-side reflective film 402 and the p-side reflective film 406, and this wavelength is an oscillation wavelength of the laser light. An integral multiple of a half of the oscillation wavelength is equal to an optical distance between reflective surfaces of the n-side reflective film 402 and the p-side reflective film 406 opposed to each other. The optical distance is a distance obtained by multiplying a distance in which light actually propagates through a certain medium by a refractive index of the medium. By applying a forward voltage between the p-side electrode 408 and the n-side electrode 409, current can be injected into the active layer 404. By the current injection, population inversion occurs in the active layer 404, and amplification of light by stimulated emission at the oscillation wavelength, that is, laser oscillation occurs. As described above, it is assumed that the p-side electrode 408 and n-side electrode 409 side of the VCSEL of the present embodiment is a mounting surface, and laser light is extracted from the semiconductor substrate 401 side.

The configuration of the VCSEL illustrated in FIG. 10 is an example. The components included in the VCSEL may be formed using known materials. The shape of some of the components included in the VCSEL may be changed, or the VCSEL may further include other components. Laser light may be extracted from the side opposite to the semiconductor substrate 401.

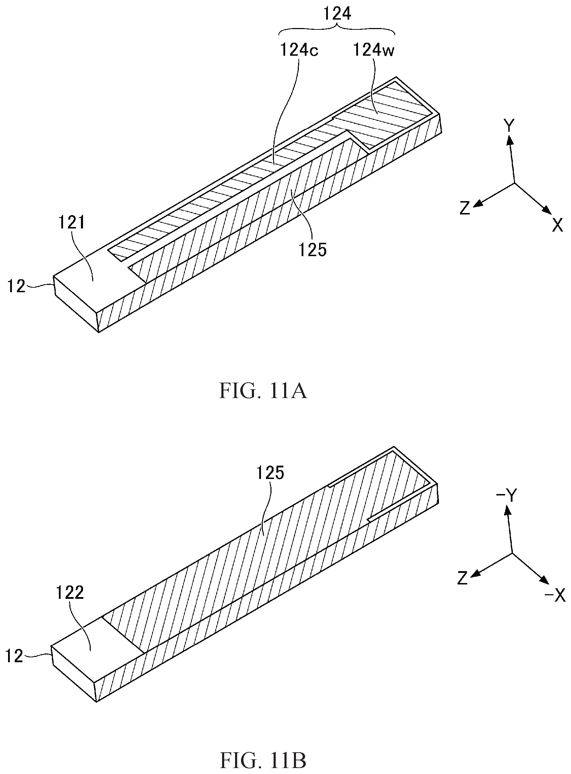

FIG. 11A is a perspective view of the support body 12 used in the third embodiment on the light-emitting element mounting surface 121 side. FIG. 11B is a perspective view of the support body on the back surface 122 side. The surface opposite to the light-emitting element mounting surface 121 is referred to as a "back surface". Conductive films 124 and 125 are formed on the light-emitting element mounting surface 121 of the support body 12. The conductive film 124 is formed only on the light-emitting element mounting surface 121 of the support body 12, and is electrically insulated from the conductive film 125. The conductive film 124 has a connection region 124c connected to one of the electrodes of the light-emitting element 31, and a wide portion 124w that is wider than the connection region 124c at the end portion of the support body 12 on the - Z side. The wide portion 124w is used for electrical connection with the insulated wire 34a. The conductive film 125 is formed from the lateral surface of the support body 12 to the back surface 122.

The p-side electrode 408 and the n-side electrode 409 of the light-emitting element 31 are connected to the conductive films 124 and 125 on the light-emitting element mounting surface 121, respectively, via the conductive connection layer (or bump). The wide portion 124w of the conductive film 124 is electrically connected to one insulated wire 34a. A part of the insulating coating on the insulated wire 34a is removed, and the inside metal wire is connected to the conductive film 124. On the back surface 122 of the support body 12, the conductive film 125 is electrically connected to the other insulated wire 34b. A part of the insulating coating on the insulated wire 34b is removed, and the inside metal wire is connected to the conductive film 125.

The insulated wires 34a and 34b are extended in the longitudinal direction (Z direction) of the support body with the support body 12 interposed between the insulated wires 34a and 34b, and do not hinder light emission from the VCSEL. In the configuration illustrated in FIG. 9, the insulated wire 34b connected to the back surface 122 of the support body 12 may be extended to the vicinity of the tip end of the support body 12 on the + Z side. The insulated wires 34a and 34b are cooled by contacting the coolant outside the case 15. The support body 12 also protrudes from at least one end portion of the case 15 in the longitudinal direction, and is cooled by the coolant. The conductive films 124 and 125 formed on the surface of the support body 12 also function as a heat sink.

The length of one side and the height of the VCSEL chip used as the light-emitting element 31 are 200 µm or less, and the VCSEL chip is mounted on the support body 12 and accommodated inside the case 15. The overall thickness and width of the light irradiation device 30 including the case 15 are about 0.5 mm, and an ultra-small light irradiation device 30 that can be incorporated into the catheter 50 is implemented.

The present disclosure has been described based on the specific configuration examples, but is not limited to the configuration examples described above. For example, in the first embodiment, the insulated wire 14b that does not hinder light emission may be extended outward from the tip end of the case 15 in the + Z direction and may function as a heat dissipation member. In the configuration of the first embodiment or the third embodiment, a photodetector such as a photodiode may be disposed together with the light-emitting element on the light-emitting element mounting surface 121 of the support body 12, and may be used as a biological sensor. In the configuration of the third embodiment, instead of mounting the two light-emitting elements 31 on the support body 12, one light-emitting element 31 and one light receiving element may be mounted. When two-core enameled wires are used as the insulated wires 14a, 14b, 34a, and 34b, one metal wire may be used for supplying an electrical signal to the light-emitting element 11 or 31, and the other metal wire may be used as a wire for reading a signal output from the photodetector.

The light irradiation devices 10, 20, and 30 of the first to third embodiments may be used in combination with an endoscope. The light-emitting elements 11 and 31 can be used not only as a laser light source for treatment, but also as a sensing light source or an illumination light source. In any of these cases, the light irradiation device has a heat dissipation property that allows heat from the light-emitting element to be dissipated to the outside, and an electrical insulation property sufficient to secure an insulation property in a coolant, and exhibits an effect as a light irradiation device that can be incorporated into a catheter. Unlike an optical fiber, an insulated wire having an insulating coating is highly flexible. In addition, the configuration in which the light-emitting element itself is mounted on the support body 12 offers high integration, and has a wide range of applications for sensors.

The temperature simulation is performed using the configuration of the second embodiment, with the support body 12 made of SiN and having a length L of 1.7 mm, two-core enameled wires as the insulated wires 24a and 24b, and a glass tube having an outer diameter of 0.5 mm and a length of 1.0 mm as the case 15. As a cooling condition, physiological saline at a temperature of 35°C is supplied at a flow rate of 1 cm/sec. When the light-emitting element 11 outputs light at 50 mW, the temperature of the center of the light-emitting element is 50°C, the temperature of the center of the support body 12 is 46°C, and the temperature of the coolant at the outlet of the catheter is 38°C. When output of the light from the light-emitting element 11 is 30 mW, the temperature of the center of the light-emitting element is 45°C, the temperature of the center of the support body 12 is 42°C, and the temperature of the coolant at the outlet of the catheter is 37°C. Practical use is highly expected.

### Industrial Applicability

The present invention can be used not only for a light irradiation device inserted into a medical catheter, but also for other medical applications. The present invention is also applicable to a sensor or the like that requires local light irradiation in combination with a coolant.

Embodiments of the present disclosure include, for example, following configurations.
[Aspect 1] A light irradiation device comprising: a light-emitting element configured to emit light having a predetermined wavelength; a support body on which the light-emitting element is mounted; insulated wires electrically connected to the light-emitting element; a case accommodating the light-emitting element, connection portions between a respective one of the insulated wires and the light-emitting element, and a part of the support body; and a sealing member having an insulation property and sealing the light-emitting element, the part of the support body, and the connection portions inside the case, wherein the other part of the support body protrudes from the sealing member toward an outside of the case.
[Aspect 2] The light irradiation device according to Aspect 1, wherein both ends of the case along a longitudinal direction of the support body are sealed by the sealing member with the light-emitting element and the connection portion accommodated inside the case, and the support body protrudes from at least one of the both ends of the case toward the outside of the case.
[Aspect 3] The light irradiation device according to Aspect 1 or 2, wherein both ends of the case along a longitudinal direction of the support body are sealed by the sealing member with the light-emitting element and the connection portion accommodated inside the case, and each of the insulated wires is extended outward from at least one of the both ends of the case to the outside of the case.
[Aspect 4] The light irradiation device according to Aspect 3, wherein the insulated wires comprise a first insulated wire electrically connected to the light-emitting element on a side of a light-emitting element mounting surface of the support body, and a second insulated wire electrically connected to the light-emitting element on a surface of the support body opposite to the light-emitting element mounting surface, and the first insulated wire and the second insulated wire are disposed with the light-emitting element and the support body located between the first insulated wire and the second insulated wire.
[Aspect 5] The light irradiation device according to Aspect 4, wherein the first insulated wire and the second insulated wire are extended outward from the both ends of the case to the outside of the case.
[Aspect 6] The light irradiation device according to Aspect 5, wherein a length of each of the first insulated wire and the second insulated wire extended outward from the both ends of the case to the outside of the case is greater on a side opposite to a light-emitting surface side of the light-emitting element than on the light-emitting surface side of the light-emitting element.
[Aspect 7] The light irradiation device according to any one of Aspects 1 to 3, wherein the light-emitting element is an edge-emitting laser element, and an optical component is disposed inside the case, the optical component being configured to guide light emitted from the edge-emitting laser element in a direction intersecting a light-emitting element mounting surface of the support body.
[Aspect 8] The light irradiation device according to Aspect 7, wherein the insulated wire comprises a first insulated wire electrically connected to the light-emitting element on a side of the light-emitting element mounting surface of the support body, and a second insulated wire electrically connected to the light-emitting element on a surface of the support body opposite to the light-emitting element mounting surface, and the first insulated wire and the second insulated wire are extended outward of the case on a side opposite to a light-emitting surface of the edge-emitting laser element.
[Aspect 9] The light irradiation device according to any one of Aspects 1 to 3, wherein the light-emitting element is an edge-emitting laser element, and an optical component is provided inside the case, the optical component being configured to cause light emitted from the edge-emitting laser element to travel in a direction parallel to a light-emitting element mounting surface of the support body.
[Aspect 10] The light irradiation device according to Aspect 9, wherein the insulated wires comprise a first insulated wire electrically connected to the light-emitting element on a side of a light-emitting element mounting surface of the support body, and a second insulated wire electrically connected to the light-emitting element on a surface of the support body opposite to the light-emitting element mounting surface, and light emitted from the edge-emitting laser element is extracted from a region between the first insulated wire and the second insulated wire to the outside of the case.
[Aspect 11] The light irradiation device according to any one of Aspects 1 to 3, wherein the light-emitting element is a vertical cavity surface emitting laser, and a light-emitting surface of the vertical cavity surface emitting laser faces the case in a direction perpendicular to a light-emitting element mounting surface of the support body.
[Aspect 12] The light irradiation device according to any one of Aspects 1 to 11, wherein one or both of the case and the sealing member are a biocompatible resin.
[Aspect 13] An in-vivo light irradiation assembly comprising: a catheter; and the light irradiation device according to any one of Aspects 1 to 12, the light irradiation device being inserted into the catheter, wherein an inside of the catheter is filled with a coolant at least during use.

This application claims priority based on Japanese Patent Application No. 2023-211659, filed with the Japan Patent Office on December 15, 2023, the entire contents of which are included herein by reference.

### Reference Characters List

10, 20, 30 Light irradiation device
11, 31 Light-emitting element
111, 317 Light-emitting surface
12, 12-1, 12-2 Support body
121 Light-emitting element mounting surface
122 Back surface
124, 125 Conductive film
14a, 14b, 24a, 24b, 34a, 34b Insulated wire
15 Case
16 Sealing material
17, 27 Optical component
171, 172 Optical element
50 Catheter
51 Coolant
100, 200 In-vivo light irradiation assembly

## Claims

1. A light irradiation device comprising:
a light-emitting element configured to emit light having a predetermined wavelength;
a support body on which the light-emitting element is mounted;
insulated wires electrically connected to the light-emitting element;
a case accommodating the light-emitting element, connection portions each between a respective one of the insulated wires and the light-emitting element, and a part of the support body; and
a sealing member having an insulation property and sealing the light-emitting element, the part of the support body, and the connection portions inside the case,
wherein the other part of the support body protrudes from the sealing member toward an outside of the case.

2. The light irradiation device according to claim 1, wherein
both ends of the case along a longitudinal direction of the support body are sealed by the sealing member with the light-emitting element and the connection portions accommodated inside the case, and
the support body protrudes from at least one of the both ends of the case toward the outside of the case.

3. The light irradiation device according to claim 1 or 2, wherein
both ends of the case along a longitudinal direction of the support body are sealed by the sealing member with the light-emitting element and the connection portions accommodated inside the case, and
each of the insulated wires is extended outward from at least one of the both ends of the case to the outside of the case.

4. The light irradiation device according to claim 3, wherein
the insulated wires comprise
a first insulated wire electrically connected to the light-emitting element on a side of a light-emitting element mounting surface of the support body, and
a second insulated wire electrically connected to the light-emitting element on a surface of the support body opposite to the light-emitting element mounting surface, and
the first insulated wire and the second insulated wire are disposed with the light-emitting element and the support body located between the first insulated wire and the second insulated wire.

5. The light irradiation device according to claim 4, wherein the first insulated wire and the second insulated wire are extended outward from the both ends of the case to the outside of the case.

6. The light irradiation device according to claim 5, wherein a length of each of the first insulated wire and the second insulated wire extended outward from the both ends of the case to the outside of the case is greater on a side opposite to a light-emitting surface side of the light-emitting element than on the light-emitting surface side of the light-emitting element.

7. The light irradiation device according to any one of claims 1 to 3, wherein
the light-emitting element is an edge-emitting laser element, and
an optical component is disposed inside the case, the optical component being configured to guide light emitted from the edge-emitting laser element in a direction intersecting a light-emitting element mounting surface of the support body.

8. The light irradiation device according to claim 7, wherein
the insulated wire comprises a first insulated wire electrically connected to the light-emitting element on a side of the light-emitting element mounting surface of the support body, and a second insulated wire electrically connected to the light-emitting element on a surface of the support body opposite to the light-emitting element mounting surface, and
the first insulated wire and the second insulated wire are extended outward of the case on a side opposite to a light-emitting surface of the edge-emitting laser element.

9. The light irradiation device according to any one of claims 1 to 3, wherein
the light-emitting element is an edge-emitting laser element, and
an optical component is provided inside the case, the optical component being configured to cause light emitted from the edge-emitting laser element to travel in a direction parallel to a light-emitting element mounting surface of the support body.

10. The light irradiation device according to claim 9, wherein
the insulated wires comprise a first insulated wire electrically connected to the light-emitting element on a side of a light-emitting element mounting surface of the support body, and a second insulated wire electrically connected to the light-emitting element on a surface of the support body opposite to the light-emitting element mounting surface, and
light emitted from the edge-emitting laser element is extracted from a region between the first insulated wire and the second insulated wire to the outside of the case.

11. The light irradiation device according to any one of claims 1 to 3, wherein
the light-emitting element is a vertical cavity surface emitting laser, and
a light-emitting surface of the vertical cavity surface emitting laser faces the case in a direction perpendicular to a light-emitting element mounting surface of the support body.

12. The light irradiation device according to any one of claims 1 to 11, wherein one or both of the case and the sealing member are a biocompatible resin.

13. An in-vivo light irradiation assembly comprising:
a catheter; and
the light irradiation device according to any one of claims 1 to 12, the light irradiation device being inserted into the catheter,
wherein an inside of the catheter is filled with a coolant at least during use.
